# EUROPÄISCHE PATENTSCHRIFT

(11) **EP 2 892 581 B1**
(45) Veröffentlichungstag und Bekanntmachung des Hinweises auf die Patenterteilung: **28.12.2016**
(21) Anmeldenummer: 13771047.1
(22) Anmeldetag: 15.08.2013
(51) Int. Cl.: A61M 1/00, A61M 27/00, A61M 25/00

(54) **MEDIZINISCHER DRAINAGESCHLAUCH**
MEDICAL DRAINAGE TUBE
TUYAU DE DRAINAGE MÉDICAL

(30) Priorität: 05.09.2012 DE 102012108248
(43) Veröffentlichungstag der Anmeldung: 15.07.2015
(73) Patentinhaber: Asskea GmbH, 99189 Gebesee (DE)
(72) Erfinder: HERTWIG, Sven, 99718 Grüningen (DE); GRAF, Marco, 99718 Greußen (DE)
(74) Vertreter: Weidner Stern Jeschke
(86) Internationale Anmeldenummer: PCT/DE2013/100295
(87) Internationale Veröffentlichungsnummer: WO 2014/036993

(56) Entgegenhaltungen:
- WO-A2-2009/071933
- US-A- 4 735 606

## Beschreibung

Die Erfindung betrifft einen medizinischen Drainageschlauch für die Behandlung von Wunden mittels von einem medizinischen Absauggerät erzeugtem Unterdruck.

### Stand der Technik

Zum Fördern des Heilungsprozesses an offenen Wunden ist es bekannt, neben dem üblichen Absaugen von Wundsekret, die Wunde kontinuierlich oder in Intervallen einem definierten Unterdruck auszusetzen. Dadurch wird bewirkt, dass das die Wunde umgebende Epithelgewebe und das subkutane Gewebe verstärkt zur Wunde wandern und dadurch ein schnellerer Wundverschluss erreicht wird.

Dazu muss die zu behandelnde Wunde so durch Nähen oder Klammern abdichtend verschlossen werden, dass durch das Anlegen einer Saugleitung ein Unterdruck an der Wunde aufgebaut werden kann. Ist insbesondere bei großflächigen Wunden ein Abdichten gegen den atmosphärischen Druck durch Nähen oder Klammern nicht möglich, erfolgt ein Abdichten durch das Abdecken der Wunde mittels Pflastern oder Wundverbänden.

Um die während des natürlichen Heilungsprozesses sowie die bei einer diese unterstützenden Unterdruckbehandlung auftretenden Sekretansammlungen abzuführen, ist es üblich, einen in der Wunde platzierten Drainageschlauch an eine medizinische Absaugvorrichtung mit Sektretauffangbehälter anzuschließen. Eine derartige Wundbehandlungsvorrichtung ist unter anderem aus EP 0 853 950 B1 bekannt. Gegenstand der darin beschriebenen Lösung ist weiterhin ein spezielles Wundverbandkissen. Um nämlich kontinuierlich Sekret aus einer Wunde in einen Sekretauffangbehälter zu fördern, muss eine gewisse Druckdifferenz zwischen dem Druck im Wundraum und dem atmosphärischem Druck, also eine gewisse Belüftung des Wundraumes gewährleistet sein. Dabei dürfen jedoch keine Keime, zum Beispiel aus der Umgebungsluft, in die Wunde gelangen. So ist es erforderlich, die der Wunde zugeführte Belüftungsluft entsprechend zu filtern.

Besonders problematisch stellt sich die Belüftung des Wundraumes dar, wenn es sich bei den zu behandelnden Wunden um körperinnere Wunden handelt oder die Wunde durch einen Wundverband luftdicht verschlossen werden muss.

Zum Absaugen von Sekret aus einer oberflächlichen, mit einer dichten chirurgischen Wundabdeckung versehenen Wunde ist aus EP 0 865 304 B1 eine tragbare Wundbehandlungsvorrichtung bekannt, bei welcher an die chirurgische Wundabdeckung eine mit zusätzlichen Lumen versehene Saugleitung angeschlossen ist. Über zusätzliche Lumen wird ermöglicht, dass der Absaugstelle Luft zugeführt wird. Das Absauggerät muss dabei speziell für die Steuerung der Luftzufuhr über das Belüftungslumen ausgebildet sein.

Zum Absaugen von Sekreten aus Körperhöhlen ist es aus DE 43 06 478 A1 bekannt, zusätzlich zu dem zum Absauggerät führenden Drainageschlauch eine Zusatzleitung vorzusehen, durch welche vorzugsweise Luft an die Absaugstelle geleitet wird. Zum Steuern dieser Luftzufuhr muss das Absauggerät ebenfalls mit entsprechender Mess-, Steuer- und Filtertechnik versehen sein. Die Verwendung eines herkömmlichen, nur mit Saugeingang versehenen medizinischen Absauggerätes ist mit dieser Drainagevorrichtung nicht möglich.

Die WO 2009/071933 A2 betrifft die Unterdrucktherapie an Wunden, welche ebenfalls eine die Wunde abdichtende Wundauflage verwendet. Dazu wird eine mit einer Unterdruckquelle verbindbare und mit einem perforierten Drain versehene Saugleitung abgedichtet durch die Wundauflage in den Wundraum geführt. Um ein Belüften des Wundraumes nahe der Saugstelle zu ermöglichen, muss eine zusätzliche Belüftungsleitung abgedichtet durch die Wundauflage geführt werden. Am Ende der Belüftungsleitung ist in einem separaten und mit einer Lufteinlassöffnung versehenen Gehäuse ein Bakterienfilter angeordnet. Dieser Bakterienfilter weist eine fest eingestellte Permeabilität auf, welche die Belüftungsintensität festlegt. Um dies bei einer angegebenen Porengröße des Bakterienfilters von 0,2 µm zu ermöglichen, muss der Bakterienfilter als relativ großflächiger Membranfilter ausgelegt werden. Durch das dazu notwendige separate Filtergehäuse in Verbindung mit der separaten Belüftungsleitung ist der Anwendungsbereich dieser Lösung sehr beschränkt. Durch das Positionieren und Abdichten von zwei separaten Leitungen entsteht insbesondere bei der Versorgung innenliegender Wunden, welche nicht durch eine Wundabdeckung verschlossen werden, ein erhöhter Aufwand. Nachteilig ist weiterhin, dass bei der durch eine Schlauchklemme abgesperrten Belüftungsleitung Feuchtigkeit durch die Lufteinlassöffnung des Filtergehäuses an den Bakterienfilter gelangen kann und dieser dann auf Grund seiner hydrophoben Eigenschaften dauerhaft unwirksam wird. Insbesondere bei mobilen Anwendungen und bei Anwendungen, bei welchen der Drainageschlauch auch bei der Benutzung von Sanitäreinrichtungen in der Wunde verbleibt, ist dies sehr nachteilig.

Die DE 10 2011 052 735 A1 beschreibt einen mehrlumigen medizinischen Drainageschlauch, bei welchem das Sauglumen am proximalen Ende in eine axiale Belüftungsöffnung mündet. Gegenstand der US 4,735,606 ist eine medizinische Drainagevorrichtung, bei welcher eine mehrlumige Drainageleitung mit ihrem distalen Ende in einer Körperhöhle platziert wird, während das proximale Ende der Drainageleitung über Auffangbehälter an eine Unterdruckquelle angeschlossen ist. Diese Drainagevorrichtung ist an eine herkömmliche, nur einen Saugeingang aufweisende Unterdruckquelle anschließbar. Problematisch ist bei dieser Lösung, dass in das auch proximal offene Belüftungslumen erst über Reibschluss dichtend eine regulierbare Belüftungs- und Filtereinheit angeschlossen werden muss. Ist diese Belüftungs- und Filtereinheit nicht oder nicht korrekt angeschlossen, können Keime aus der Umgebungsluft in die zu behandelnde Wunde gelangen. Obwohl auch diese medizinische Drainagevorrichtung an übliche, nur mit Saugeingang versehene medizinische Absauggeräte anschließbar ist, kann sie durch die Art der Belüftung der Absaugstelle hinsichtlich Handhabung, konstruktivem Aufbau und Sicherheit nicht befriedigen. Durch das Erfordernis, die Intensität der Belüftung situationsabhängig mittels eines einstellbaren Ventils über den beschriebenen weiten Regelbereich zu beeinflussen, muss der verwendete Filter eine Permeabilität und damit eine Porengröße aufweisen, die den heutigen Anforderungen an Bakterienfilter nicht gerecht werden.

Die DE 31 27 249 A1 betrifft eine chirurgische Drainagevorrichtung mit einem Adapterstück von einem einlumigen auf einen dreilumigen Schlauch mit axialer Belüftungsöffnung. Durch den vor dem Gebrauch notwendigen Zusammenbau ist die Keimfreiheit nicht gewährleistet.

Die DE 43 06 478 A1 offenbart eine Drainagevorrichtung, bei welcher ein patientenseitig mehrlumiger Saugschlauch am proximalen Ende in zwei getrennte einlumige Schlauchabschnitte übergeht, wobei im separaten Schlauchabschnitt der Zusatzleitung ein Bakterienfilter eingebunden ist.

Die bekannten technischen Lösungen sind hinsichtlich Kompaktheit und Handhabung nicht zufriedenstellend. Insbesondere für die Anwendung bei solchen Weichteildrainagen, bei denen das proximale Ende eines einzigen Saugschlauches mit einem Spieß neben der Wundöffnung von innen nach außen durch die Gewebeschichten gezogen wird, sind diese Lösungen nicht geeignet.

### Aufgabe

Es ist daher Aufgabe der Erfindung, eine Lösung für einen kompakten, im konstruktiven Aufbau sehr einfachen medizinischen Drainageschlauch mit integriertem Belüftungslumen zum Absaugen von Sekret aus Wunden oder Körperhohlräumen zu schaffen, mit welcher ein Eindringen von Keimen aus der Umgebungsluft über das Belüftungslumen zuverlässig verhindert wird. Die Absaugvorrichtung muss an übliche, nur mit Saugeingang versehene medizinische Absauggeräte anschließbar sein. Außerdem soll der erfindungsgemäße Drainageschlauch auch bei Weichteildrainagen, bei denen das proximale Ende des mehrlumigen Saugschlauches mit einem Spieß neben der Wundöffnung von innen nach außen durch die jeweiligen Gewebeschichten gezogen wird, anwendbar sein.

Diese Aufgabe wird durch einen durchgehend einstückigen Drainageschlauch mit mindestens einem Sauglumen und mindestens einem Belüftungslumen gelöst, wobei das distale Ende des Sauglumens am Umfang perforiert ausgebildet ist und das proximale Ende des Sauglumens an eine Unterdruckquelle anschließbar ist. Dabei führt vom Belüftungslumen, von der proximalen Mündung deutlich beabstandet, eine radiale Belüftungsöffnung nach außen und kommuniziert mit einem, in einer den Saugschlauch koaxial umschließenden Belüftungsmuffe unlösbar angeordneten Bakterienfilter. Dieser ist in seiner Permeabilität so auf die Unterdruckquellen herkömmlicher medizinischer Absauggeräte abgestimmt, dass bei offener proximaler Mündung des Belüftungslumens eine definierte Druckdifferenz zwischen dem Sauglumen und dem Belüftungslumen gewährleistet wird.

Vorteilhaft ist dabei, dass die zum Bakterienfilter führende Lufteinlassöffnung in der Belüftungsmuffe zum distalen Ende des Drainageschlauches gerichtet ist. Im Zusammenhang mit der vom proximalen Ende des Saugschlauches deutlich beabstandeten Anordnung der Belüftungsmuffe ist die zum Bakterienfilter führende Lufteinlassöffnung somit nicht in der Nähe des Sauganschlusses des Drainageschlauches. Dadurch kann beim Hantieren am Sauganschluss des Drainageschlauches, auch bei nicht in der Belüftungsmuffe eingesetztem Verschlussstopfen, keine Feuchtigkeit in den Bakterienfilter gelangen.

In proximaler Richtung weist die Belüftungsmuffe vor dem Bakterienfilter eine Abschrägung auf, wodurch das Hindurchführen durch die jeweiligen Gewebeschichten erleichtert wird.

Der Aufbau der Belüftungsmuffe, bestehend aus zwei zu verbindenden Halbschalen, in Verbindung mit Klemmwulsten sowie einer Abquetschwulst, ermöglicht ein kostengünstiges Herstellen der Belüftungsmuffe sowie deren einfaches Anbringen am Saugschlauch.

Ist eine Belüftung der Behandlungsstelle über das Belüftungslumen nicht erwünscht, wird die zum Bakterienfilter führende Lufteinlassöffnung durch einen Verschlussstopfen dicht verschlossen.

Ein weiterer wesentlicher Vorteil der Erfindung besteht darin, dass durch die im Luftweg zum Belüftungslumen unlösbar integrierte Anordnung des Bakterienfilters jederzeit ein Eindringen von Keimen über das Belüftungslumen verhindert wird. Durch das Abstimmen der Permeabilität des Bakterienfilters auf die Unterdruckquellen herkömmlicher medizinischer Wundabsauggeräte erübrigen sich zusätzliche Mittel zum Einstellen der optimalen Druckdifferenz zwischen Sauglumen/Wunde und Belüftungslumen. Die erfindungsgemäße Lösung für das Zuschalten und Abschalten des Belüftungslumens und die Einstellung der Druckdifferenz ermöglicht eine sehr kompakte, zubehörarme Gestaltung des Drainageschlauches, was insbesondere beim Einsatz tragbarer medizinischer Wundabsauggeräte vorteilhaft ist.

Die Verwendung eines Pipettenfilters mit einer Porengröße von 3 - 12 µm gestattet eine vorteilhafte und platzsparende Anordnung in der Anschlusseinheit. Besonders vorteilhaft ist das Zuschalten und Abschalten des Sauglumens über einen unverlierbar an der Belüftungsmuffe angeordneten Verschlussstopfen, welcher die Lufteinlassöffnung in der Belüftungsmuffe schon vor dem Bakterienfilter verschließt. Dadurch kann insbesondere bei Anwendungen, bei denen der Drainageschlauch, zum Beispiel bei der Benutzung von Sanitäranlagen durch den Patienten, in der Wunde verbleibt, keine Feuchtigkeit in den Bakterienfilter eindringen.

Die erfindungsgemäße Absaugvorrichtung ist für Unterdruckwundbehandlungen mit und ohne Sekretabführung einsetzbar.

### Ausführungsbeispiel

Es zeigen:
- Fig. 1: einen Drainageschlauch im Längsschnitt
- Fig. 2: den Schnitt A-A durch das distale Ende eines Drainageschlauches
- Fig. 3: den axialen Schnitt durch eine Belüftungsmuffe ohne Saugschlauch
- Fig. 4: die Einzelheit X aus Fig. 1 als axialen Schnitt

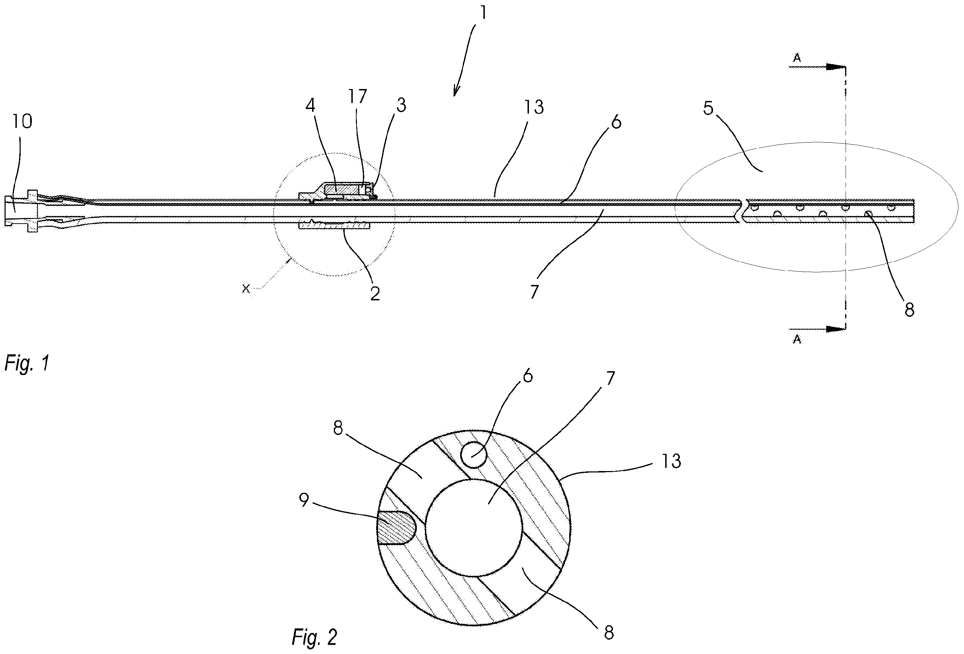

Im dargestellten Ausführungsbeispiel nach den Figuren 1 bis 4 weist der erfindungsgemäße Drainageschlauch 1 einen einstückigen, flexiblen und zweilumigen Saugschlauch 13 auf. Der Saugschlauch 13 besteht aus dem Sauglumen 7 und dem Belüftungslumen 6. Am distalen Ende des Saugschlauches 13 weist das Sauglumen 7 eine Perforation 8 auf. Das Belüftungslumen 6 mündet unperforiert am distalen Ende des Saugschlauches 13. In der Wandung des Saugschlauches 13 verläuft der Röntgenkontraststreifen 9 (Fig. 2).

Am proximalen Ende des Saugschlauches 13 mündet das Sauglumen 7 in den Sauganschluss 10, während das Belüftungslumen 6 vorteilhaft an einer flanschförmigen Fläche des Sauganschlusses 10 endet.

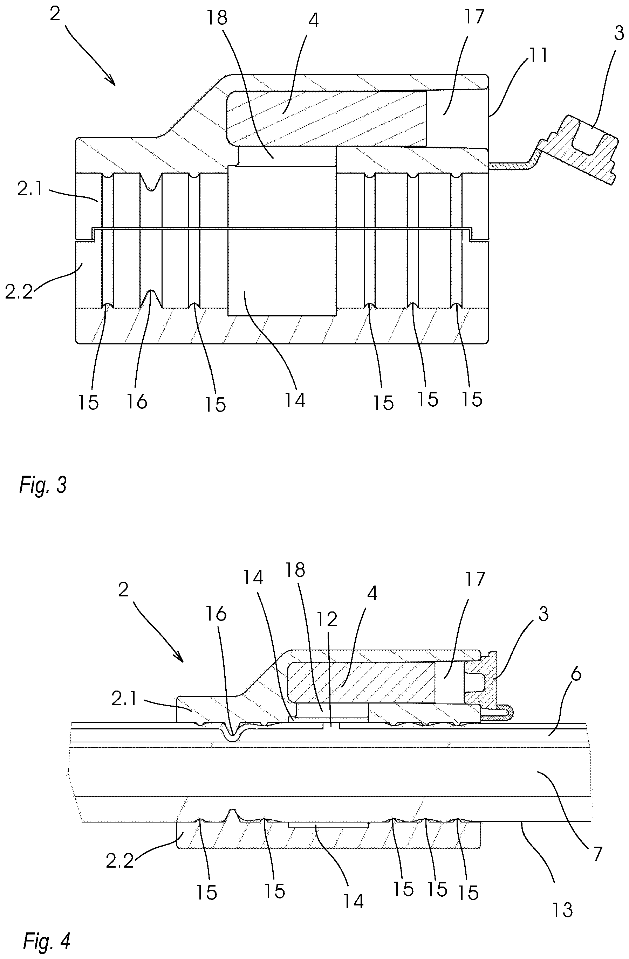

Vom Sauganschluss 10 deutlich beabstandet weist die Außenwandung des Belüftungslumens 6 eine radiale Belüftungsöffnung 12 auf. An dieser Stelle ist, den Saugschlauch 13 koaxial umschließend, die Belüftungsmuffe 2 angeordnet, welche aus verschweißten oder verklebten Halbschalen 2a und 2b besteht (Fig. 3).

In der Wandung der Halbschale 2a ist in einem Filterraum 17 der Bakterienfilter 4 angeordnet. Im Bereich der Belüftungsöffnung 12 ist in der Innenwandung der Belüftungsmuffe 2, also in den Halbschalen 2a und 2b, eine Ringnut 14 ausgebildet, in welche die Belüftungsöffnung 12 mündet. Nach außen ist die Ringnut 14 über den Durchbruch 18 mit dem Filterraum 17 verbunden. Die in distaler Richtung angeordnete Lufteinlassöffnung 11 ist durch einen Verschlussstopfen 3 verschließbar. Beidseitig des Ringraumes 14 sind an der Innenwandung der Belüftungsmuffe 2 umlaufend die Klemmwulste 15 ausgebildet, durch welche die Belüftungsmuffe 2 den Saugschlauch 13 dicht umschließend fixiert wird. In proximaler Richtung neben der Belüftungsöffnung 12 ist an der Innenwandung der Belüftungsmuffe 2 umlaufend die Quetschwulst 16 ausgebildet. Diese ist radial so dimensioniert, dass sie nur das Belüftungslumen 6 an dieser Stelle dicht verschließend abquetscht. Das zum distalen Ende des Saugschlauches führende Belüftungslumen 6 kann somit bei entferntem Verschlussstopfen 3 über die Ringnut 14, die Belüftungsöffnung 12, die Ringnut 14, den Durchbruch 18 und den Bakterienfilter 4 saugend mit der Atmosphäre in Wirkverbindung stehen.

Zwischen der Belüftungsmuffe 2 und dem perforierten Bereich des zweilumigen Saugschlauches 13 kann bedarfsweise eine nicht dargestellte Schlauchklemme angebracht werden.

Um im Bereich der Wundhöhle 5 einen definierten Unterdruck zu erzeugen, wird der Saugschlauch 13 zumindest mit seinem perforierten distalen Ende in der Wundhöhle 5 platziert. Dazu kann der perforierte Bereich des Saugschlauches 13 situationsabhängig gekürzt werden. An seinem unperforierten Umfang wird der Saugschlauch 13 gegenüber dem die Wundhöhle 5 verschließenden Gewebe oder einer Wundabdeckung abgedichtet. Durch die zweilumige Ausbildung des Saugschlauches 13 sind diese Abdichtungen nur an einer Stelle vorzunehmen.

Zum Anlegen des Unterdrucks in der Wundhöhle 5 wird an den Sauganschluss 10 eine nicht dargestellte medizinische Wundabsaugvorrichtung angeschlossen. Beim Betrieb der Wundabsaugvorrichtung wird sich in der Wundhöhle 5 ansammelndes Sekret über die distale Mündung des Sauglumens 7 sowie dessen Perforation 8 angesaugt und in den Sammelbehälters des nicht dargestellten medizinischen Wundabsauggerätes befördert. Um bei einer abgedichteten Wundhöhle den Sekretfluss aufrechtzuerhalten, ist die Lufteinlassöffnung 11 nicht durch den Verschlussstopfen 3 verschlossen. Durch den von der Lufteinlassöffnung 11 an die distale Mündung des Belüftungslumen 6 gelangenden Luftstrom wird die Absaugstelle in optimaler Weise belüftet. Die Kapazität des Luftdurchflusses ist fest eingestellt und auf die jeweilig verwendeten medizinischen Wundabsauggeräte abgestimmt. Diese feste Einstellung des Luftdurchflusses wird durch den jeweils verwendeten Bakterienfilter 4 realisiert. Vorteilhaft weist der Bakterienfilter 4 dazu eine Porengröße von 3 - 12 µm auf. Bei der sich aus dieser Porengröße ergebenden Permeabilität ist der Bakterienfilter 4 vorteilhaft als Pipettenfilter mit einer Länge von ca. 3 - 6 mm und einem Durchmesser von ca. 2,5 mm realisierbar. Diese geometrischen Abmessungen erlauben vorteilhaft das unlösbare Integrieren des Bakterienfilters 4 in die Wandung der Belüftungsmuffe 2. Dies ist eine Voraussetzung für die kompakte und zubehörarme Gestaltung des erfindungsgemäßen Drainageschlauches 1.

Soll auf eine Belüftung an der Absaugstelle verzichtet werden, zum Beispiel bei oberflächlichen Wunden oder bei der Verwendung von Wundabdeckungen aus Gaze, Schwämmen oder semipermeablen Folien, wird die Lufteinlassöffnung 11 mit dem an der Belüftungsmuffe 2 ausgebildeten Verschlussstopfen 3 dicht verschlossen und damit der Luftstrom im Belüftungslumen 6 unterbrochen. In diesem Zustand kann auch keine Feuchtigkeit in den hydrophoben Bakterienfilter 4 eindringen und diesen dauerhaft funktionsunfähig machen. Mit eingesetztem Verschlusstopfen 3 kann sich der Patient zum Beispiel mit eingeführtem Drainageschlauch 1 einer erhöhten Feuchtigkeit in Sanitärräumen aussetzen.

Zum Sichtbarmachen des Saugschlauches 13 auf Röntgenaufnahmen dient der in dessen Wandung verlaufende Röntgenkontraststreifen 9.

Die Ausbildung des Sauganschlusses 10 als 4 mm Standardanschluss gewährleistet die Adaptierbarkeit des erfindungsgemäßen Drainageschlauches 1 an alle gängigen medizinischen Saugsysteme.

### Bezugszeichenliste

- 1: Drainageschlauch
- 2: Belüftungsmuffe
- 3: Verschlussstopfen
- 4: Bakterienfilter
- 5: Wundhöhle
- 6: Belüftungslumen
- 7: Sauglumen
- 8: Perforation
- 9: Röntgenkontrastreifen
- 10: Sauganschluss
- 11: Lufteinlassöffnung
- 12: Belüftungsöffnung
- 13: Saugschlauch
- 14: Ringnut
- 15: Klemmwulst
- 16: Quetschwulst
- 17: Filterraum
- 18: Durchbruch

## Patentansprüche

1. Medizinischer Drainageschlauch (1) für die Behandlung von Wunden mittels von einem medizinischen Absauggerät erzeugtem Unterdruck, wobei der Drainageschlauch (1) mehrlumig ausgebildet ist und einen Saugschlauch (13) mit mindestens einem Sauglumen (7) und mindestens einem Belüftungslumen (6) aufweist, **dadurch gekennzeichnet, dass** die Außenwandung des Belüftungslumens (6), von einem Sauganschluss (10) beabstandet, eine radiale Belüftungsöffnung (12) aufweist und an dieser Stelle, den Saugschlauch (13) koaxial dicht umschließend, eine Belüftungsmuffe (2) angeordnet ist, in deren Innenwandung, mit der Belüftungsöffnung (12) kommunizierend, eine Ringnut (14) ausgebildet ist, welche wiederum mit einem in einem Filterraum (17) in der Wandung der Belüftungsmuffe (2) unlösbar angeordneten Bakterienfilter (4) kommuniziert, welcher über eine Lufteinlassöffnung (11) mit der Atmosphäre in Verbindung steht, wobei der vom Ringraum (14) zum proximalen Ende des Saugschlauches (13) führende Teil des Belüftungslumens (6) abgedichtet ist.

2. Medizinischer Drainageschlauch nach Patentanspruch 1, **dadurch gekennzeichnet, dass** die Belüftungsmuffe (2) aus zwei zusammengefügten Halbschalen (2a, 2b) besteht, wobei axial beidseitig des Ringraumes (14) an der Innenwandung der Belüftungsmuffe (2) umlaufende Klemmwulste (15) ausgebildet sind.

3. Medizinischer Drainageschlauch nach Patentanspruch 1 oder 2, **dadurch gekennzeichnet, dass** in proximaler Richtung neben der Belüftungsöffnung (12) umlaufend eine das Belüftungslumen (6) abdichtende Quetschwulst (16) ausgebildet ist.

4. Medizinischer Drainageschlauch nach einem der Patentansprüche 1 bis 4, **dadurch gekennzeichnet, dass** die Lufteinlassöffnung (11) zum distalen Ende des Drainageschlauches (1) gerichtet und durch einen Verschlussstopfen (3) verschließbar ist.

## Claims

1. A medical drainage tube (1) for treatment of wounds by means of a negative pressure generated by a medical suction apparatus, wherein the drainage tube (1) is formed with multiple lumen and has a suction tube (13) with at least one suction lumen (7) and at least one ventilation lumen (6), **characterized in that** the outer wall of the ventilation lumen (6), has a radial ventilation opening (12) which is spaced-apart from a suction connector (10), and that a ventilation sleeve (2) is disposed **in that** place in such a manner that it coaxially tightly surrounds the suction tube (13), an annular groove (14), which communicates with the ventilation opening (12), being disposed in the inner wall of said ventilation sleeve, said annular groove communicating in turn with a bacterial filter (4) undetachably disposed in a filter compartment (17) in the wall of the ventilation sleeve (2), said bacterial filter being connected to the atmosphere by way of an air inlet opening (11), wherein the part of the ventilation lumen (6) leading from the annular space (14) to the proximal end of the suction tube (13) is sealed off.

2. The medical drainage tube according to claim 1, **characterized in that** the ventilation sleeve (2) consists of two assembled half-shells (2a, 2b), wherein, on both axial sides of the annular space (14), clamping beads (15) are circumferentially formed on the inner wall of the ventilation sleeve (2).

3. The medical drainage tube according to claim 1 or 2, **characterized in that** a squeezing bead (16) sealing off the ventilation lumen (6) is circumferentially formed next to the ventilation opening (12) in the proximal direction.

4. The medical drainage tube according to one of the claims 1 to 4, **characterized in that** the air inlet opening (11) is oriented toward the distal end of the drainage tube (1) and is closable by means of a stopper (3).

## Revendications

1. Tuyau de drainage médical (1) pour le traitement de plaies au moyen d'une dépression générée par un appareil d'aspiration médical, où le tuyau de drainage
(1) est réalisé avec plusieurs lumières et comporte un tuyau d'aspiration (13) avec au moins une lumière d'aspiration (7) et au moins une lumière d'aération (6), **caractérisé en ce que** la paroi extérieure de la lumière d'aération (6) comporte une ouverture d'aération radiale (12) à distance d'un raccord d'aération (10), et qu'un manchon d'aération (22) est disposé à cet endroit de telle manière qu'il entoure coaxialement et hermétiquement le tuyau d'aspiration (13), une gorge annulaire (14) en communication avec l'ouverture d'aération (12) étant formée dans la paroi intérieure dudit manchon d'aération, ladite gorge d'aération communicant à son tour avec un filtre à bactéries (4) disposé de manière inamovible dans un espace de filtrage (17) dans la paroi du manchon d'aération
(2) et relié à l'atmosphère via une ouverture d'admission d'air (11), où la partie de la lumière d'aération (6) s'étendant de l'espace annulaire (14) jusqu'à l'extrémité proximale du tuyau d'aération (13) est hermétiquement fermée.

2. Tuyau de drainage médical selon la revendication 1, **caractérisé en ce que** le manchon d'aération (2) est composé de deux demi-coques (2a, 2b) assemblées, où des bourrelets de serrage (15) sont formés axialement de part et d'autre de l'espace annulaire (14) sur le pourtour de la paroi intérieure du manchon d'aération (2).

3. Tuyau de drainage médical selon la revendication 1 ou 2, **caractérisé en ce qu'**un bourrelet de sertissage (16) fermant hermétiquement la lumière d'aération (6) est formé sur le pourtour, à côté de l'ouverture d'aération (12) dans la direction proximale.

4. Tuyau de drainage médical selon l'une des revendications 1 à 4, **caractérisé en ce que** l'ouverture d'admission d'air (11) est orientée vers l'extrémité distale du tuyau de drainage (1) et peut être fermée par un bouchon de fermeture (3).
